# EUROPEAN PATENT SPECIFICATION

(11) **EP 0 754 686 B1**
(45) Date of publication and mention of the grant of the patent: **15.09.1999**
(21) Application number: 96401619.0
(22) Date of filing: 19.07.1996
(51) Int. Cl.: C07D 241/04

(54) **2-cyanopiperazine and use thereof for the synthesis of biologically active substances**
2-Cyanopiperazin und ihre Verwendung für die Synthese von biologisch wirksamen Stoffen
2-Cyanopipérazine et son usage pour la synthèse de substances biologiquement actives

(30) Priority: 19.07.1995 JP 18285795; 12.10.1995 JP 26405995; 12.10.1995 JP 26406095
(43) Date of publication of application: 22.01.1997
(73) Proprietor: Mitsubishi Chemical Corporation, Chiyoda-ku, Tokyo 100 (JP)
(72) Inventor: Takuma, Yuki, c/o Mitsubishi Chem. Corp., Yahata-ku, Kitakyusyu-shi, Fukuoka (JP); Kasuga, Yuzo, c/o Mitsubishi Chem. Corp., Yahata-ku, Kitakyusyu-shi, Fukuoka (JP); Miyazaki, Takeki, c/o Mitsubishi Chem. Corp., Yahata-ku, Kitakyusyu-shi, Fukuoka (JP); Mizuho, Yuji, c/o Mitsubishi Chem. Corp., Yahata-ku, Kitakyusyu-shi, Fukuoka (JP); Okamoto, Ken, c/o Mitsubishi Chem. Corp., Yahata-ku, Kitakyusyu-shi, Fukuoka (JP); Murakami, Takeshi, c/o Mitsubishi Chem. Corp., Yahata-ku, Kitakyusyu-shi, Fukuoka (JP)
(74) Representative: Uchida, Kenji

(56) References cited:
- EP-A- 0 710 652
- CHEMICAL ABSTRACTS, vol. 111, no. 25, 1989 Columbus, Ohio, US; abstract no. 232862w, page 798; column 1; XP002014971 & JP-A-01 117 869 (HOKURIKU PHARMA.) 10 May 1989

## Description

The present invention relates to 2-cyanopiperazine represented by the following formula (I): 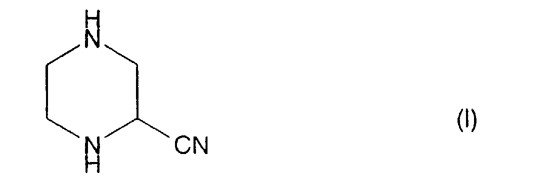 and a production method thereof. 2-cyanopiperazine is a novel compound and is useful as an intermediate for medicaments, agricultural chemicals, etc.

Also, the present invention relates to N-tert-butyl-2-piperazine carboxamide, derived from the 2-cyanopiperazine represented by the formula (I) as starting material. The optically active N-tert-butyl-2-piperazine carboxamide is also a useful compound as an intermediate for medicaments, for example, as an intermediate for the preparation of the HIV protease inhibitor "indinavir".

(S)-N-tert-butyl-2-piperazine carboxamide represented by the following formula (II): 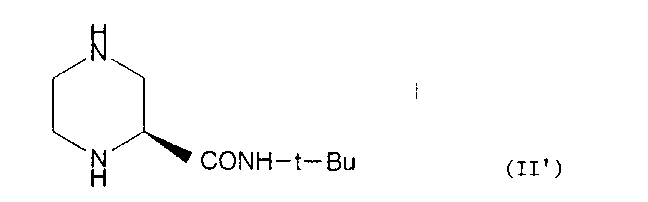 is a precursor of chiral (S)-N-Boc-piperazine MK-639 of the formula (III) 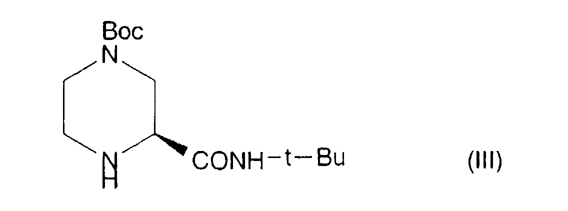 where Boc represents a tert-butoxycarbonyl group.

This product (III) is an intermediate product for preparing the HIV protease inhibitor "indinavir".

Merck HIV protease inhibitor "indinavir" represented by the following formula (V) is described in Tetrahedron Letters, volume 36, pages 6419-6422, 1995. This inhibitor is constructed by coupling an epoxide represented by the following formula (IV) with (S)-2-tert-butyl carboxamide-4-tert-butoxycarbonylpiperazine represented by the formula (III), as shown in the following reaction diagram:

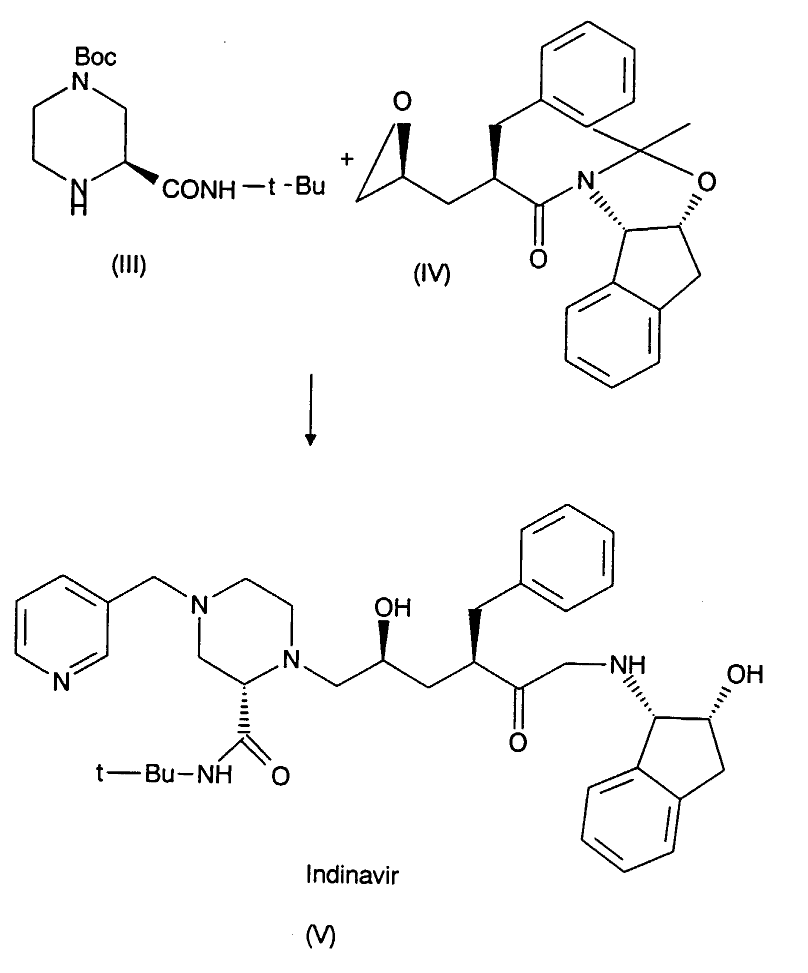

(S)-2-tert-butylcarboxamide-4-tert-butoxycarbonylpiperazine represented by the formula (III) is obtained by the following procedure.

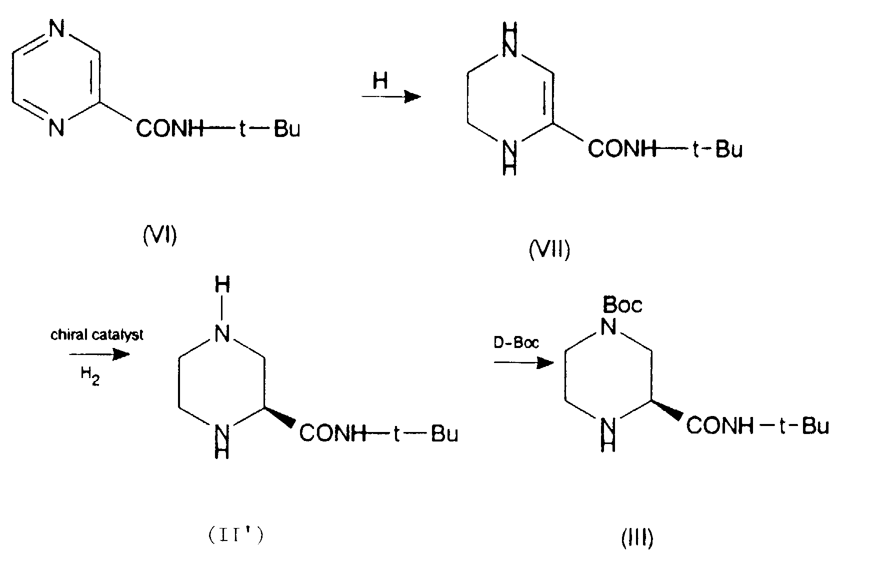

In this diagram, D-Boc represents di-tert-butoxy-dicarbonate.

N-tert-butyl-2-pyrazine carboxamide represented by the formula (VI) is obtained by reacting cyanopyrazine represented by the following formula (VIII) with tert-butyl alcohol in the presence of sulfonic acid (see JP-A-7-145153, the term "JP-A-" as used herein means an unexamined published Japanese patent application).

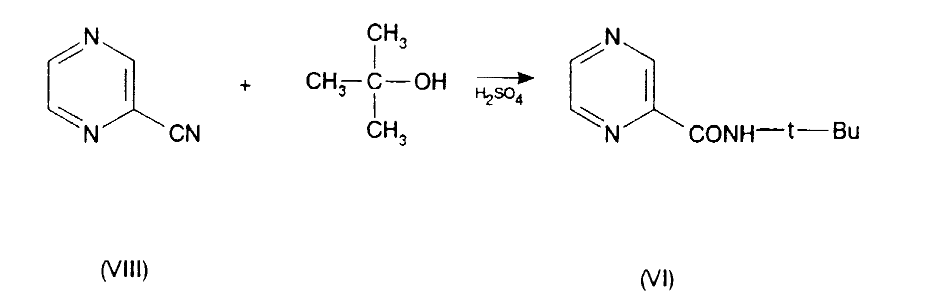

Also, hitherto, in regard to the production method of N-tert-butyl-2-piperazine carboxamide represented by preceding formula (II), the only method consists of hydroreducing N-tert-butyl-2-pyrazine carboxamide represented by the following formula (VI) in the presence of a platinum oxide catalyst or an LiAlH₄ catalyst under the conditions of 50°C and 100 atmospheric pressure as described in JP-A-1-117869.

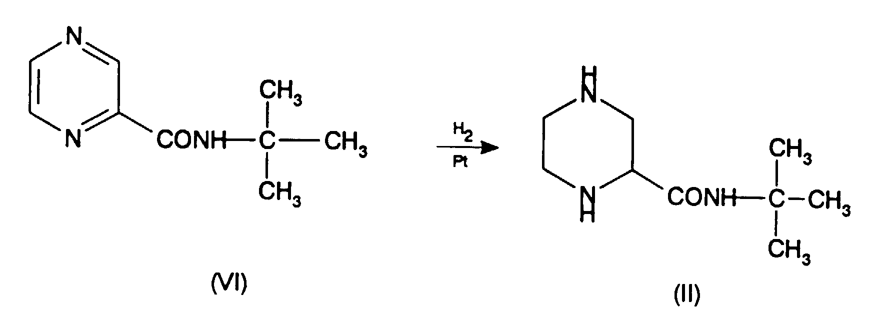

However, the foregoing method has an industrial disadvantage since, in this method, an expensive platinum oxide catalyst is required and also high-pressure equipment enduring 100 atmospheric pressure is required. Hence a method of producing N-tert-butyl-2-piperazine carboxamide with low cost and with good yield has been desired.

It has now been discovered that N-tert-butyl-2-piperazine carboxamide represented by the formula (II) described above can be produced without using such an expensive catalyst, by using instead 2-cyanopiperazine of the present invention as the starting material.

Thus, an object of the present invention is to provide the novel compound, 2-cyanopiperazine represented by the formula (I) described above, which is useful as an intermediate for medicaments, agricultural chemicals, etc.

Another object of the present invention is to provide a method of producing 2-cyanopiperazine represented by the formula (I).

Another object of the present invention is to provide N-tert-butyl-2-piperazine carboxamide represented by the formula (II) described above, which is a useful intermediate for preparation of the HIV protease inhibitor "indinavir", obtained from 2-cyanopiperazine represented by the formula (I) of the present invention.

To this end, there is provided a method of preparing an optically active form of N-tert-butyl-2-piperazine carboxamide having the formula (II).

The method comprises the steps of:
a) providing 2-cyanopiperazine of formula (I);
b) reacting said 2-cyanopiperazine (I) with tert-butyl alcohol in the presence of an acid, thereby obtaining the racemic form of N-tert-butyl-2-piperazine carboxamide (II); and,
c) optically resolving said racemic form into an optically active form of N-tert-butyl-2-piperazine carboxamide (II) in the presence of an optically resolving agent.

In particular, the method may be used to prepare (S) form of N-tert-butyl-2-piperazine carboxamide (II) and this (S) form may further be processed to prepare an HIV protease inhibitor.

As one aspect of the invention, the optical resolution in step c) may be effected by using an optically active N-acyl amino acid.

This optically active N-acyl amino acid is preferably either N-benzyloxycarbonyl-L-phenylalanine or N-benzyloxycarbonyl-L-aspartic acid.

An optically active N-acyl amino acid can more generally be used as an agent for optically resolving a racemic compound.

To enable the above method, 2-cyanopiperazine of the formula (I) or a salt thereof is specifically prepared.

This compound may be prepared by reacting a 2-halogenoacrylonitrile having the formula (IX) 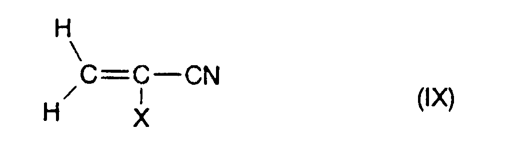 where X is a halogen atom; with ethylenediamine.

The molar proportion of ethylenediamine to 2-halogenoacrylonitrile (IX) is preferably about 1.5 : 1.

In a preferred embodiment, the 2-halogenoacrylonitrile (IX) comprises 2-chloroacrylonitrile.

The compound may also be prepared by reacting a 2,3-dihalogenopropionitrile having the formula (X): 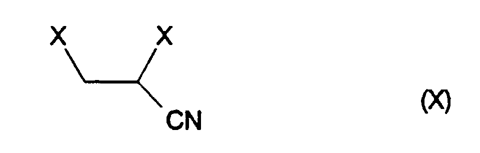 where X is a halogen atom; with ethylenediamine.

The molar proportion of ethylenediamine to 2,3-dihalogenopropio-nitrile is preferably about 2 : 1.

In a preferred embodiment, 2,3-dihalogenopropionitrile (X) comprises 2,3-dichloropropionitrile.

Where appropriate, the reaction is effected in the presence of a base. Also, the reaction can be effected in the presence of a solvent.

As is apparent from the above, 2-cyanopiperazine of the formula (I) can be used for the prepartion of N-tert-butyl-2-piperazine carboxamide of the formula (II) which is a useful intermediate product for a biologically active substance, in particular, an HIV protease inhibitor.

According to the present invention, 2-cyanopiperazine or a salt thereof, useful as a starting material for medicaments, agricultural chemicals, etc., can be easily produced, and also by using 2-cyanopiperazine, N-tert-butyl-2-piperazine carboxamide can be produced at a low cost and with a good yield.

Also, according to the method of this invention, by using an inexpensive material as a resolving agent, the optical resolution of N-tert-butyl-2-piperazine carboxamide as the racemic mixture (RS) can be carried out by a simple operation and with a high yield.

2-cyanopiperazine of this invention represented by the formula (I) is prepared by reacting a 2-halogenoacrylonitrile represented by the formula (IX) and ethylenediamine, as follows.

The reaction can be carried out without solvent but is preferably carried out in an inert or relatively inert solvent for the purpose of the invention.

As such solvents, ethers such as tetrahydrofuran, diethyl ether, dibutyl ether, etc.; alcohols such as methyl alcohol, ethyl alcohol, butyl alcohol, etc.; (halo)aromatic hydrocarbons such as benzene, toluene, xylene, chlorobenzene, dichlorobenzene, etc; aliphatic or alicyclic hydrocarbons such as hexane, heptane, cyclohexane, etc.; halogenated hydrocarbons such as dichloromethane, dichloroethane, chloroform, carbon tetrachloride, etc.; acetonitrile; water; etc., are used. Preferably, tetrahydrofuran, toluene, dichloromethane, dichloroethane, chloroform, etc., are used. The solvent is usually used in an amount 1 to 20 times by volume the amount of the 2-halogenoacrylonitrile.

When the reaction of this invention is carried out without solvent, it is preferred to use an excessive amount of ethylenediamine so that a part of ethylenediamine functions as a solvent.

Since hydrohalogenic acid is formed during the reaction, a base is added thereto to capture the acid. As the base, amines such as triethylamine, pyridine, dimethylaniline, etc., sodium hydroxide, potassium hydroxide, sodium carbonate, etc. are used and the amount of the base to be used is from 1 to 3 molar equivalents, preferably from 1 to 2 molar equivalents to one mole of the 2-halogenoacrylonitrile.

In a preferred embodiment of the present invention. ethylenediamine is used excessively so that a part of ethylenediamine functions as a base. The amount of ethylenediamine to be used is usually determined in the range of from 1 to 10 moles per mole of the 2-halogenoacrylonitrile, depending on the presence or absence of a base and a solvent being used together. When a solvent is used but a base is not used together with ethylenediamine, it is preferred to use from 1.5 to 2 moles of ethylenediamine to one mole of the 2-halogenoacrylonitrile.

The reaction of the 2-halogenoacrylonitrile and ethylenediamine is usually carried out at a temperature of from -20°C to +100°C but from the point of view of the reaction rate and yield, it is preferred to carry out the reaction at a temperature of from 20°C to 60°C.

The reaction pressure may be such that the reaction system keeps its liquid phase. The reaction is usually carried out at normal pressure, but, if desired, the reaction may be carried out under a reduced pressure or under positive pressure.

The reaction can be most simply carried out by charging an inert solvent, ethylenediamine, and a base in a reaction vessel and adding dropwise the 2-halogenoacrylonitrile to the mixture under stirring.

Since the reaction is exothermic, the reaction system may be cooled where appropriate.

The separation and the purification of 2-cyanopiperazine thus formed from the reaction miuxture can be carried out by any conventional separation and purification methods. For example, after by-products are precipitated from the reaction mixture as salts and removed by filtration, an acid is added to the filtrate to precipitate 2-cyanopiperazine as an acid addition salt, which is collected by filtration. As the acid to be added in this case, an inorganic acid such as hydrochloric acid, sulfuric acid, hydrobromic acid, etc., is preferred and the amount of the acid is from 1 to 3 molar equivalents, preferably from 1 to 1.5 molar equivalent to one mole of 2-cyanopiperazine.

The acid addition salt of 2-cyanopiperazine is dissolved in water to form an aqueous solution thereof, then by neutralizing the aqueous solution with sodium hydroxide or the like, 2-cyanopiperazine is liberated. By extracting the product with an organic solvent such as ethyl acetate or the like and then removing the organic solvent by evaporation, 2-cyanopiperazine can be isolated.

Also, 2-cyanopiperazine is produced with high yield by reacting a 2,3-dihalogenopropionitrile represented by the formula (III) described above and ethylenediamine. In the formula (III), X is preferably chlorine, bromine or iodine.

The reaction can be carried out without solvent but is preferably carried out in an inert or relatively inert solvent. As the inert solvent, such solvents used in the reaction described above can be similarly used. Among these inert solvents, tetrahydrofuran, toluene, dichloromethane, dichloroethane, chloroform, etc., are preferably used. The solvent is usually used in an amount 1 to 20 times by volume the amount of the 2,3-dihalogenopropionitrile.

When the reaction is carried out without solvent, it is preferred that ethylenediamine is excessively used so that a part of ethylenediamine functions as solvent.

Since hydrohalogenic acid is formed during the reaction, a base is added thereto to capture the acid. As the base, amines such as triethylamine, pyridine, dimethylaniline, etc., sodium hydroxide, potassium hydroxide, sodium carbonate, etc. are used and the amount of the base to be used is from 2 to 4 molar equivalents, preferably from 2 to 3 molar equivalents, to one mole of the 2,3-dihalogenopropionitrile.

In a preferred embodiment of the present invention, ethylenediamine, a starting material, is added in excess, so that a part thereof functions as the base. Usually the amount of ethylenediamine to be used is determined in the range from 1 to 20 moles per mole of the 2,3-dihalogenopropionitrile, depending on the presence or absence of the base and the solvent being used. When a solvent is used but the base is not used together with ethylenediamine, it is preferred to use from 2 to 10 moles of ethylenediamine per mole of the 2,3-dihalogenopropionitrile.

The reaction of the 2,3-dihalogenopropionitrile and ethylenediamine is usually carried out at a temperature of from -20°C to +100°C but from the point of view of the reaction rate and yield, it is preferred to carry out the reaction at a temperature of from 20°C to 60°C.

The reaction pressure may be such that the reaction system keeps the liquid phase. Usually the reaction is carried out at normal pressure but, if desired, the reaction may be carried out under a reduced pressure or under positive pressure.

The reaction can be most simply carried out by charging an inert solvent, ethylenediamine, and a base in a reaction vessel and adding dropwise the 2,3-dihalogenopropionitrile to the mixture under stirring. Since the reaction is exothermic, the reaction system is cooled where appropriate.

For separating and purifying 2-cyanopiperazine from the reaction mixture, the same separation and purification method as described in the production of 2-cyanopiperazine can be used.

For producing N-tert-butyl-2-piperazine carboxamide of formula (II), 2-cyanopiperazine of formula (I) is reacted with tert-butyl alcohol in the presence of an acid, as shown in the following reaction scheme:

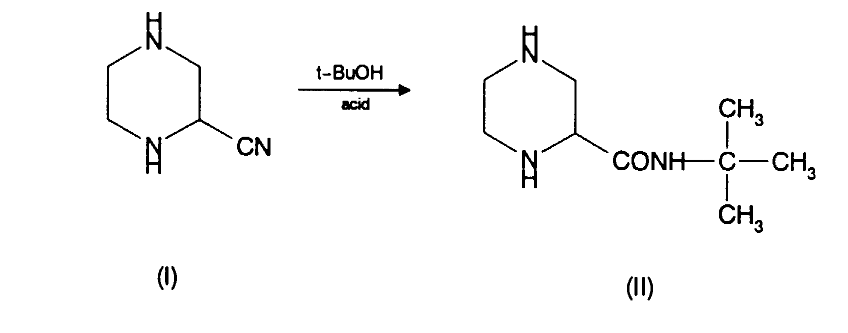

From a practical point of view, the reaction is carried out either by adding dropwise tert-butyl alcohol to the mixture of an acid and 2-cyanopiperazine, or by adding dropwise 2-cyanopiperazine to the mixture of an acid and tert-butyl alcohol, or by adding dropwise an acid to the mixture of 2-cyanopiperazine and tert-butyl alcohol.

As the acid, sulfuric acid, orthophosphoric acid, formic acid, Lewis acid, etc., can be used. In particular, sulfuric acid is preferred.

In the case of using sulfuric acid, the reation may proceed using diluted sulfuric acid of about 10% in concentration but the use of sulfuric acid of a concentration from 50% to 90% is preferred in order to obtain a high yield. The amount of the acid to be used is preferably at least the theoretical amount necessary for neutralizing the amino group of 2-cyanopiperazine and decomposing the cyano group of 2-cyanopiperazine by the addition of alcohol.

For example, in the case of sulfuric acid, the amount to be used is at least 2 moles, preferably 2 to 20 moles, more preferably still 5 to 15 moles per mole of 2-cyanopiperazine, to obtain the desired product with high yield. When the amount of sulfuric acid to be used is less than 2 moles to one mole of 2-cyanopiperazine, the product yield is low.

Also, it is preferred that the amount of tert-butyl alcohol to be used is at least on the equimolar basis with regard to 2-cyanopiperazine. From an economical point of view, the appropriate amount is from about 1 to 2 moles.

The reaction temperature is usually from 0°C to 80°C, and preferably from 0°C to 50°C.

In each of the foregoing reactions, 2-cyanopiperazine, tert-butyl alcohol, or an acid is added dropwise at an above-mentioned temperature during a period of 0.5 to 5 hours. After having kept the reaction system for a period of 0 to 10 hours at the same temperature, the reaction is completed.

Then, N-tert-butyl-2-piperazine carboxamide formed by the reaction can be isolated by neutralizing the reaction mixture by the addition of an alkali, extracting the reaction mixture with an organic solvent, and then removing the organic solvent from the extract by distillation. Also, in another method, N-tert-butyl-2-piperazine carboxamide can be obtained by adding dropwise the reaction mixture to a water-soluble organic solvent and precipitating N-tert-butyl-2-piperazine carboxamide as an acid addition salt thereof.

Usually, to obtain optically active N-tert-butyl-2-piperazine carboxamide of the formula (II) by optically resolving the racemic form of the carboxamide, two diastereomers are formed into salts with various kinds of optically active α-oxycarboxylic acids, then, the diasteromer salts formed are resolved by virtue of the difference of their solubility. For example, a method of using optically active lactic acid, malic acid, or tartaric acid as a resolving agent is described in JP-A-8-3145.

According to another aspect of the invention, an optically active N-acylamino acid is used to resolve racemic N-tert-butyl-2-piperazine carboxamide into its optically active form with a higher yield.

Examples of such an optically active N-acylamino acid type resolving agent are N-benzyloxycarbonyl-L-phenylalanine (hereinafter referred to as Z-L-Phe) and N-benzyloxycarbonyl-L-aspartic acid (hereinafter referred to as Z-L-Asp). Either L-form or D-form of these optically active N-acylamino acids can be used to effect an optical resolution.

The amount of optically active N-acylamino acid to be used is usually from 0.5 to 1.5 moles, preferably on an equimolar basis with regards to racemic N-tert-butyl-2-piperazine carboxamide.

For operating the optical resolution, a solvent is usually used. There is no particular restriction for the solvent to be used, if it can dissolve both racemic N-tert-butyl-2-piperazine carboxamide and the optically active N-acylamino acid without modifying their nature, and can preferably precipitate that one of the two kinds of diasteromer salts formed which is less soluble in the solvent. As such a solvent, water, a hydrophilic organic solvent like alcohols such as methanol, ethanol, n-propyl alcohol, isopropyl alcohol, etc.; ketones such as acetone, methyl ethyl ketone, etc.; ethers such as tetrahydrofuran, dioxane, etc.; acetonitrile, and N,N-dimethylformamide, or a mixture thereof is preferred.

The temperature at which the optically active N-acylamino acid and racemic N-tert-butyl-2-piperazine carboxamide are mixed may not be higher than the boiling point of the solvent. It is usually in the range from 0°C to 100°C or preferably from 0°C to 80°C. The temperature of the crystallization is preferably not higher than 50°C to obtain a high yield.

The diasteromer salt obtained by the crystallization is, if necessary, separated and purified by the method of recrystallization, reprecipitation, etc., whereby the optical purity of N-tert-butyl-2-piperazine carboxamide contained in the diasteromer salt mixture can be further increased.

After obtaining the diasteromer salt as described above, this salt is decomposed by an appropriate method, to isolate optically active N-tert-butyl-2-piperazine carboxamide and the resolving agent. For example, after an aqueous solution containing the diasteromer salt is alkalified, optically active N-tert-butyl-2-piperazine carboxamide is extracted with water and a hydrophobic organic solvent, then the solvent phase is separated and distilled off, whereby optically active N-tert-butyl-2-piperazine carboxamide can be obtained.

The hydrophobic organic solvent to be used in the above process preferably does not hinder the completion of the reaction and dissolves N-tert-butyl-2-piperazine carboxamide. Suitable examples of the hydrophobic organic solvent are halogenated hydrocarbons such as methylene chloride, chloroform, dichloroethane, carbon tetrachloride, etc.; aromatic hydrocarbons such as benzene, toluene, xylene, etc.; aliphatic hydrocarbons such as hexane, heptane, cyclohexane, etc.; esters such as ethyl formate, ethyl acetate, propyl acetate, etc.; and ethers such as diethyl ether, dibutyl ether, etc.

As the base to be used, there are, for example, alkali metal hydroxides such as sodium hydroxide, potassium hydroxide, barium hydroxide, lithium hydroxide, etc.; alkali metal carbonates such as sodium carbonate, potassium carbonate, etc.; alkali metal hydrogencarbonates such as sodium hydrogencarbonate, potassium hydrogencarbonate, lithium hydrogencarbonate, etc.

The decomposition reaction is carried out in the range between 0°C and the reflux temperature. However at high temperatures, hydrolysis or racemization of the amide may occur. Therefore, the reaction is preferably carried out at a temperature ranging from 0°C to 20°C.

After extracting optically active N-tert-butyl-2-piperazine carboxamide, the resulting aqueous layer is acidified and extracted with an appropriate water-insoluble solvent. The solvent is then distilled off. An optically active N-acylamino acid can thus be recovered and reused as the resolving agent.

The optically active N-tert-butyl-2-piperazine carboxamide can further be purified through recrystallization, reprecipitation, or by applying to optically active column chromatography, to increase the optical purity thereof.

The above and other objects, features and advantages of the invention will be made apparent from the following description of the preferred embodiments, given as a non-limiting example.

### EXAMPLE 1

Into 300 ml of tetrahydrofuran were added 54.1 g (0.9 mole) of ethylenediamine and the mixture was heated to 30°C. To the mixture were added dropwise 52.5 g (0.6 mole) of 2-chloroacrylonitrile over a period of 2 hours with stirring and further stirring was performed for 6 hours. During stirring, the temperature was kept at about 30°C. The reaction mixture formed was cooled to 20°C and precipitates formed were removed by filtration. After adding 35% hydrochloric acid to the filtrate formed to adjust the pH of the mixture to 4, precipitates formed were collected by filtration. The precipitates were dissolved in 20% hydrochloric acid and the solution thus formed was added dropwise to tetrahydrofuran. Precipitates formed were collected by filtration and the solvent entrained in the precipitates was removed under reduced pressure to provide 55.2 g (yield 50%) of 2-cyanopiperazine dihydrochloride. The analytical values of the product were as follows:
1H-NMR (D₂O, 400 MHz): δ (ppm): 3.2 to 3.4 (4H, m), 3.57 (2H, dd, J = 12.0 and 4.0 Hz), 4.72 (1H, t, J = 4.0 Hz)
IR (KBr): ν(cm⁻¹): 3400, 2905, 2655, 2409, 2143, 1529, 1430, 1340, 1290, 1071, 940, 558, 519

| Elemental Analysis: | | | |
|---|---|---|---|
| | C | H | N |
| Calculated: | 32.6% | 6.0% | 22.8% |
| Found: | 32.6% | 6.2% | 22.6% |

### EXAMPLE 2

In Example 1, after the precipitates were filtered off from the reaction mixture, 10% sulfuric acid was added to the filtrate to adjust the pH of the mixture to 4. Precipitates formed were then collected by filtration. The precipitates collected were dissolved in 10% sulfuric acid and the solution was added dropwise to tetrahydrofuran. Precipitates formed were collected by filtration and the solvent entrained in the precipitates was removed under reduced pressure to provide 57.7 g (yield 46%) of 2-cyanopiperazine sulfate. The analytical values of the product were as follows:
1H-NMR (D₂O, 400 MHz): δ(ppm): 3.1 to 3.3 (4H, m), 3.47 (2H, dd, J = 16.0 and 4.0 Hz), 4.51 (1H, t, J = 4.0 Hz)
IR (KBr): ν(cm⁻¹) : 3405, 2935, 2590, 2460, 2135, 1602, 1477, 1439, 1137, 1098, 1060, 1015, 614

| Elemental Analysis: | | | |
|---|---|---|---|
| | C | H | N |
| Calculated: | 28.7% | 5.3% | 20.1% |
| Found: | 28.5% | 5.5% | 19.9% |

### EXAMPLE 3

Into 400 ml of tetrahydrofuran were added 180.3 g (3.0 moles) of ethylenediamine and the mixture was heated to 30°C. To the mixture were added dropwise 52.5 g (0.6 mole) of 2-chloroacrylonitrile over a period of 2 hours with stirring and further stirring was performed for 6 hours. During stirring, the temperature was kept at about 30°C. After the reaction, the reaction liquid was analyzed by liquid chromatography. Yield of 2-cyanopiperazine was 80%. Then, after distilling off tetrahydrofuran under reduced pressure, 30% sulfuric acid was added to the residue to adjust the pH to 4. Precipitates formed were collected by filtration. Furthermore, the precipitates thus collected were washed with water, filtrated, and dried to provide 88.4 g (yield 67%, purity 95%) of 2-cyanopiperazine dihydrochloride.

### EXAMPLE 4

The same procedure as in Example 3 was carried out by using 72.1 g (1.2 mole) of ethylenediamine. After the reaction, the reaction liquid was analyzed by liquid chromatography. Yield of 2-cyanopiperazine was 60%.

### EXAMPLE 5

The same procedure as in Example 3 was carried out using 360.6 g (6.0 moles) of ethylenediamine. After the reaction, the reaction liquid was analyzed by liquid chromatography. Yield of 2-cyanopiperazine was 65%.

### EXAMPLE 6

Into 300 ml of tetrahydrofuran were added 72.1 g (1.2 mole) of ethylenediamine and the mixture was heated to 30°C. To the mixture were added dropwise 73.2 g (0.6 mole) of 2,3-dichloropropionitrile over a period of 2 hours with stirring and further stirring was performed for 6 hours. During stirring, the temperature was kept at about 30°C. The reaction mixture was cooled to 20°C and precipitates formed were removed by filtration. To the filtrate was added 10% sulfuric acid to adjust the pH thereof to 4, and precipitates formed were collected by filtration. The precipitates were dissolved in 10% sulfuric acid and the solution formed wad added dropwise to tetrahydrofuran. Precipitates formed were collected by filtration and the solvent entrained in the precipitates was removed under reduced pressure to provide 57.7 g (yield 46%) of 2-cyanopiperazine sulfate. The analytical values of the product were as follows:
1H-NMR (D₂O, 400 MHz): δ(ppm): 3.1 to 3.3 (4H, m), 3.47 (2H, dd, J = 16.0 and 4.0 Hz), 4.51 (1H, t, J = 4.0 Hz)
IR (KBr): ν(cm⁻¹): 3405, 2935, 2590, 2460, 2135, 1602, 1477, 1439, 1137, 1098, 1060, 1015, 614

| Elemental Analysis: | | | |
|---|---|---|---|
| | C | H | N |
| Calculated: | 28.7% | 5.3% | 20.1% |
| Found: | 28.5% | 5.5% | 19.9% |

### EXAMPLE 7

Into 500 ml of tetrahydrofuran were added 180.3 g (3.0 moles) of ethylenediamine and the mixture was heated to 30°C. To the mixture were added dropwise 74.4 g (0.6 mole) of 2,3-dichloropropionitrile over a period of 2 hours with stirring and further stirring was effected for 5 hours. During stirring, the temperature was kept at about 30°C. After removing tetrahydrofuran from the reaction mixture under reduced pressure, the residue was neutralized with an aqueous 30% sulfuric acid solution until the pH became 1 and precipitates formed were collected by filtration. After suspending the precipitates thus collected in 500 ml of water, the suspension was filtered again and the residue obtained by the filtration was dried to provide 81.5 g (yield 67%) of 2-cyanopiperazine sulfate.

### EXAMPLE 8

By following the same procedure as in Example 7 using 360.6 g (6.0 moles) of ethylenediamine, 75.2 g (yield 60%) of 2-cyanopiperazine sulfate were obtained.

### EXAMPLE 9

Into 300 ml of tetrahydrofuran were added 54.1 g (0.9 mole) of ethylenediamine and the mixture was heated to 30°C. To the mixture were added dropwise 52.5 g (0.6 mole) of 2-chloroacrylonitrile over a period of 2 hours with stirring and further stirring was effected for 6 hours.

During stirring, the temperature was kept at about 30°C. The reaction mixture was cooled to 20°C and precipitates formed were removed by filtration. After adding 10% sulfuric acid to the filtrate formed to adjust the pH thereof to 4, precipitates formed were collected by filtration. The precipitates were dissolved in 10% sulfuric acid and the solution was added dropwise to tetrahydrofuran. Precipitates formed were collected by filtration and the solvent entrained in the precipitates was removed under reduced pressure to provide 57.7 g (yield 46%) of 2-cyanopiperazine sulfate. The analytical values of the product were as follows:
1H-NMR (D₂O, 400 MHz): δ(ppm): 3.1 to 3.3 (4H, m), 3.47 (2H, dd, J = 16.0 and 4.0 Hz), 4.51 (1H, t, J = 4.0 Hz)
IR (KBr): ν(cm⁻¹) : 3405, 2935, 2590, 2460, 2135, 1602, 1477, 1439, 1137, 1098, 1060, 1015, 614

| Elemental Analysis: | | | |
|---|---|---|---|
| | C | H | N |
| Calculated: | 28.7% | 5.3% | 20.1% |
| Found: | 28.5% | 5.5% | 19.9% |

### EXAMPLE 10

In 1029 g (8.40 moles) of an aqueous 80% sulfuric acid solution were dissolved 219.5 g (1.05 mole) of 2-cyanopiperazine sulfate with stirring while keeping the temperature below 40°C. Then, while maintaining the temperature at from 9°C to 15°C, 187 g (2.52 moles) of tert-butyl alcohol were added dropwise to the solution over a period of 3 hours and the mixture was then kept at the same temperature for 2.5 hours to complete the reaction. Then, after adding 3 liters of water to the reaction liquid thus obtained, the mixture was neutralized with an aqueous solution of 25% sodium hydroxide until the pH thereof became 10. Then, the reaction liquid was extracted twice with one liter each of chloroform, the extracts were combined, and chloroform was removed from the mixed extract under reduced pressure to provide 106.8 g (yield 55%) of N-tert-butyl-2-piperazine carboxamide having a melting point of from 151°C to 152°C.

### EXAMPLE 11

In a reaction vessel were charged 343 g of water and 389 g (5.25 moles) of tert-butyl alcohol and then 219.5 g (1.05 moles) of 2-cyanopiperazine sulfate were dissolved in the mixture with stirring while keeping the temperature below 25°C. Then, while maintaining the temperature at 25°C, 1372 g (8.4 moles) of an aqueous 80% sulfuric acid solution were added dropwise to the solution and the temperature of the mixture was then kept at 40°C for 3 hours to complete the reaction. Then, after cooling the reaction liquid to 25°C, 2700 g of tetrahydrofuran were added to the reaction liquid and the mixture was kept at 0°C for 3 hours. Precipitates were collected by filtration and dried to yield 241 g of product. The precipitates thus obtained were dissolved in 480 g of water and the solution was neutralized with an aqueous solution of 25% sodium hydroxide until the pH thereof became 13. The solution was extracted thrice with one liter each of chloroform, the extracts were combined and chloroform was removed from the mixture extract under reduced pressure to provide 142 g (yield 73%) of N-tert-butyl-2-piperazine carboxyamide.

### EXAMPLE 12

In a reaction vessel were charged 515 g of water and 93.4 g (1.26 mole) of tert-butyl alcohol and 219.5 g (1.05 mole) of 2-cyanopiperazine sulfate were dissolved in the mixture with stirring while keeping the temperature below 25°C. Then, while maintaining the temperature at 25°C, 1544 g (12.6 moles) of an aqueous 80% sulfuruc acid solution were added dropwise to the solution and the mixture was kept at 35°C for 6 hours to complete the reaction. After cooling the reaction liquid to 25°C, 1750 g of tetrahydrofuran were added to the reaction liquid and the mixture was kept at 0°C for 3 hours. Precipitates formed were collected by filtration and dried to yield 233 g of product. The precipitate thus obtained was dissolved in 900 g of water and the solution was neutralized with an aqueous solution of 47% sodium hydroxide until the pH thereof became 13. The solution was extracted four times with one liter each of chloroform, the extracts were combined, and the chloroform was removed from the mixed extract under reduced pressure to provide 142 g (yield 73%) of N-tert-butyl-2-piperazine carboxamide.

### EXAMPLE 13

After adding 35 ml of ethanol to a mixture of 0.50 g (2.70 mmoles) of N-tert-butyl-2-piperazine carboxamide as the racemic mixture and 0.81 g (2.70 mmoles) of N-benzyloxycarbonyl-L-phenylalanine (Z-L-Phe), the mixture was heated to 75°C to form a homogenous solution and then the solution was gradually cooled to 20°C over a period of 3 hours. Crystals precipitated were collected by suction filtration and washed with a small amount of ethanol. The crystals were dried under reduced pressure, to obtain 0.20 g (yield 30%) of the product.

Then, 160 mg of the crystals were dissolved in 0.50 ml of an aqueous solution of 1N sodium hydroxide, and the solution was extracted thrice with 2.0 ml each of chloroform. After distilling off chloroform from the mixed extract, the residue was dried under reduced pressure to provide 53 mg (yield 87%) of optically active N-tert-butyl-2-piperazine carboxamide.

HPLC analysis confirmed that the optical purity of the product was 84% e.e. (S-form). In addition, the optical purity of the N-tert-butyl-2-piperazine carboxamide was analyzed using an optically active HPLC column (SUMICHIRAL OA-5000, trade name, manufactured by Sumitomo Chemical Company, Limited). This analytical method was also applied for the following examples.

### EXAMPLE 14

To a mixture of 0.50 g (2.70 mmoles) of N-tert-butyl-2-piperazine carboxamide as racemic mixture and 0.72 g (2.70 mmoles) of N-benzyloxycarbonyl-L-aspartic acid (Z-L-Asp) were added 9.0 ml of ethanol to form a homogenous solution and the solution was allowed to stand for 2 hours at 20°C. Crystals precipitated were collected by suction filtration and washed with a small amount of ethanol. The crystals were dried under reduced pressure to obtain 0.56 g (yield 92%) of the product.

Then, 500 mg of the crystals were dissolved in 2.5 ml of an aqueous solution of 1N sodium hydroxide and the solution was extracted thrice with 3.0 ml each of chloroform. After distilling off chloroform from the mixed extract, the residue was dried under reduced pressure to provide 167 mg (yield 81%) of optically active N-tert-butyl-2-piperazine carboxamide. The analytical result by HPLC showed that the optical purity thereof was 74% e.e. (S-form).

## Claims

1. Method of preparing an optically active form of N-tert-butyl-2-piperazine carboxamide having formula (II): 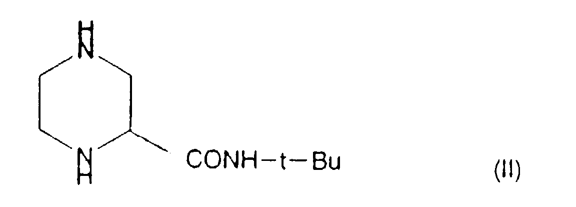 characterised in that said method comprises the steps of
a) providing 2-cyanopiperazine of formula (I) 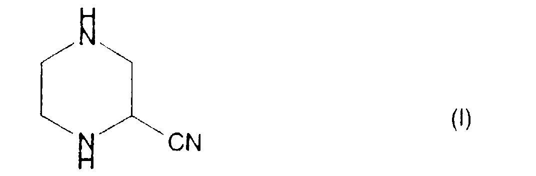
b) reacting said 2-cyanopiperazine (I) with tert-butyl alcohol in the presence of an acid, thereby obtaining the racemic form of N-tert-butyl-2-piperazine carboxamide (II); and
c) optically resolving said racemic form of N-tert-butyl-2-piperazine carboxamide (II) into an optically active form in the presence of an optically resolving agent.

2. Method according to claim 1, wherein said optically active form of N-tert-butyl-2-piperazine carboxamide (II) is (S) form.

3. Method according to claim 2, further comprising the step of incorporating said (S) form of N-tert-butyl-2-piperazine carboxamide into an HIV protease inhibitor.

4. Method according to any one of claims 1 to 3, wherein the optical resolution of step c) is effected by an optically active N-acyl amino acid.

5. Method according to claim 4, wherein said optically active N-acyl amino acid is either N-benzyloxycarbonyl-L-phenylalanine or N-benzyloxycarbonyl-L-aspartic acid.

6. 2-cyanopiperazine of the formula (I) 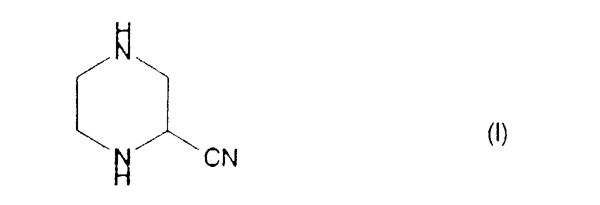 or a salt thereof.

7. Method of preparing 2-cyanopiperazine of the formula (I) defined in claim 6, characterised in that a 2-halogenoacrylonitrile having the formula (IX): 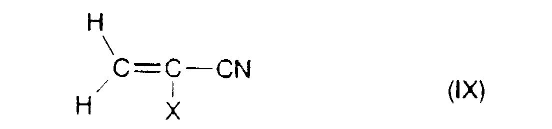 where X is a halogen atom; is reacted with ethylenediamine.

8. Method according to claim 7, wherein the molar proportion of ethylenediamine to 2-halogenoacrylonitrile (IX) is about 1.5 : 1.

9. Method according to claim 7 or 8, wherein said 2-halogenoacrylonitrile (IX) comprises 2-chloroacrylonitrile.

10. Method of preparing 2-cyanopiperazine of the formula (I) defined in claim 6, characterised in that a 2,3-dihalogenopropionitrile having the fomula (X): 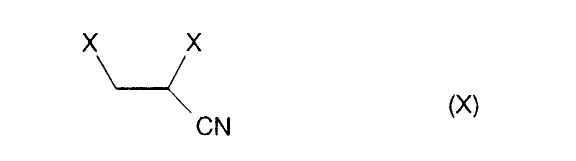 where X is a halogen atom; is reacted with ethylenediamine.

11. Method according to claim 10 ,wherein the molar proportion of ethylenediamine to 2,3-dihalogenopropionitrile is about 2 : 1.

12. Method according to claim 10 or 11, wherein said 2,3-dihalogenopropionitrile (X) comprises 2,3-dichloropropionitrile.

13. Method according to any one of claims 7 to 12, wherein the reaction is effected in the presence of a base.

14. Method according to any one of claims 7 to 13, wherein the reaction is effected in the presence of a solvent.

15. Use of 2-cyanopiperazine of the formula (I) defined in claim 6, for the preparation of N-tert-butyl-2-piperazine carboxamide of the formula (II) defined in claim 1.

16. Use of 2-cyanopiperazine of the formula (I) defined in claim 6, for the preparation of a biologically active substance.

17. Use of 2-cyanopiperazine of the formula (I) defined in claim 6 for the preparation of an HIV protease inhibitor.

## Patentansprüche

1. Verfahren zur Herstellung einer optisch aktiven Form von N-tert-Butyl-2-piperazincarboxamid mit der Formel (II): 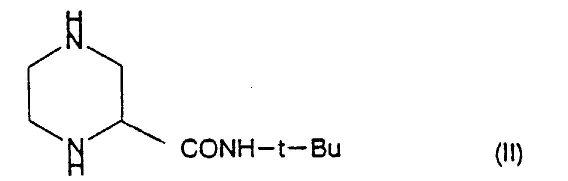 **dadurch gekennzeichnet,** daß das Verfahren die Schritte umfaßt:
a) Bereitstellung von 2-Cyanopiperazin der Formel (I) 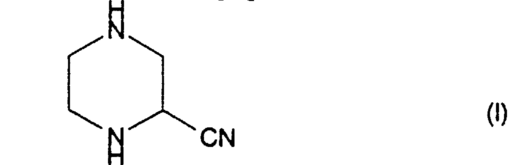
b) Umsetzung des 2-Cyanopiperazins (I) mit tert-Butylalkohol in der Anwesenheit einer Säure, hierdurch erhaltend die racemische Form von N-tert-Butyl-2-piperazincarboxamid (II); und
c) optischer Trennung der racemischen Form von N-tert-Butyl-2-piperazincarboxamid (II) in eine optisch aktive Form in der Anwesenheit eines optischen Trennungsmittels.

2. Verfahren nach Anspruch 1, **dadurch gekennzeichnet,** daß die optisch aktive Form von N-tert-Butyl-2-piperazincarboxamid (II) die (S)-Form ist.

3. Verfahren nach Anspruch 2, des weiteren umfassend den Schritt der Inkorporierung der (S)-Form von N-tert-Butyl-2-piperazincarboxamid in einen HIV-Proteaseinhibitor.

4. Verfahren nach einem der Ansprüche 1 bis 3, **dadurch gekennzeichnet,** daß die optische Trennung des Schrittes c) durch eine optisch aktive N-Acylaminosäure bewirkt wird.

5. Verfahren nach Anspruch 4, **dadurch gekennzeichnet,** daß die optisch aktive N-Acylaminosäure entweder N-Benzyloxycarbonyl-L-phenylalanin oder N-Benzyloxycarbonyl-L-asparaginsäure ist.

6. 2-Cyanopiperazin der Formel (I) 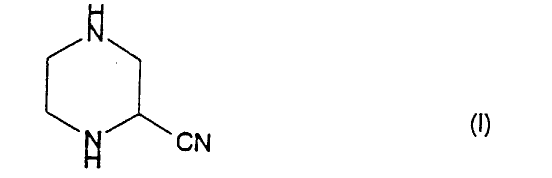 oder ein Salz desselben.

7. Verfahren zur Herstellung von 2-Cyanopiperazin der in Anspruch 6 definierten Formel (I), **dadurch gekennzeichnet,** daß ein 2-Halogenacrylnitril mit der Formel (IX): 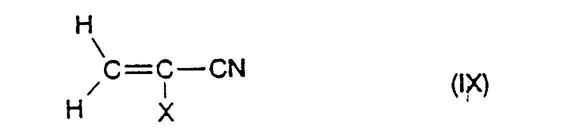 wobei X ein Halogenatom ist, mit Ethylendiamin umgesetzt wird.

8. Verfahren nach Anspruch 7, **dadurch ge kennzeichnet,** daß das molare Verhältnis von Ethylendiamin zu 2-Halogenacrylnitril (IX) ungefähr 1.5:1 ist.

9. Verfahren nach Anspruch 7 oder 8, **dadurch gekennzeichnet,** daß das 2-Halogenacrylnitril (IX) 2-Chloracrylnitril umfaßt.

10. Verfahren zur Herstellung von 2-Cyanopiperazin nach der in Anspruch 6 definierten Formel (I), **dadurch gekennzeichnet,** daß ein 2,3-Dihalogenpropionitril mit der Formel (X): 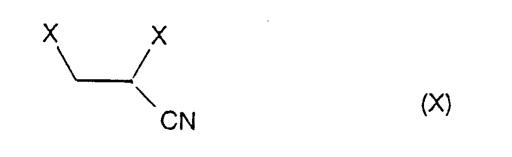 wobei X ein Halogenatom ist, mit Ethylendiamin umgesetzt wird.

11. Verfahren nach Anspruch 10, **dadurch gekennzeichnet,** daß das molare Verhältnis von Ehtylendiamin zu 2,3-Dihalogenpropionitril ungefähr 2:1 ist.

12. Verfahren nach Anspruch 10 oder 11, **dadurch gekennzeichnet,** daß das 2,3-Dihalogenpropionitril (X) 2,3-Dichlorpropionitril umfaßt.

13. Verfahren nach einem der Ansprüche 7 bis 12, **dadurch gekennzeichnet,** daß die Reaktion in Gegenwart einer Base durchgeführt wird.

14. Verfahren nach einem der Ansprüche 7 bis 13, **dadurch gekennzeichnet,**daß die Reaktion in Gegenwart eines Lösungsmittels durchgeführt wird.

15. Verwendung von 2-Cyanopiperazin nach der in Anspruch 6 definierten Formel (I) zur Herstellung von N-tert-Butyl-2-piperazincarboxamid nach der in Anspruch 1 definierten Formel (II).

16. Verwendung von 2-Cyanopiperazin nach der in Anspruch 6 definierten Formel (I) zur Herstellung eines biologisch aktiven Stoffes.

17. Verwendung von 2-Cyanopiperazin nach der in Anspruch 6 definierten Formel (I) zur Herstellung eines HIV-Proteaseinhibitors.

## Revendications

1. Procédé de préparation d'une forme optiquement active du N-tert-butyl-2-pipérazinecarboxamide de formule (II) : 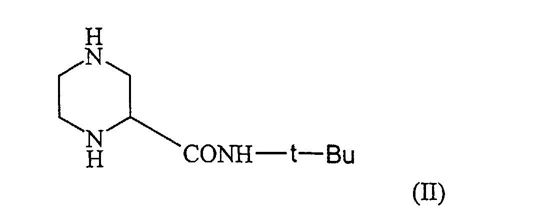 caractérisé en ce que ledit procédé comprend les étapes de
(a) fourniture de la 2-cyanopipérazine de formule (I): 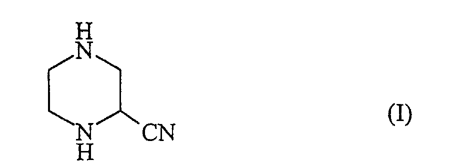
(b) réaction de ladite 2-cyanopipérazine (I) avec l'alcool tert-butylique en présence d'un acide, pour obtenir la forme racémique du N-tert-butyl-2-pipérazinecarboxamide (II) ; et
(c) résolution optique de ladite forme racémique du N-tert-butyl-2-pipérazinecarboxamide (II) en une forme optiquement active en présence d'un agent de résolution optique.

2. Procédé selon la revendication 1, dans lequel ladite forme optiquement active du N-tert-butyl-2-pipérazinecarboxamide (II) est la forme (S).

3. Procédé selon la revendication 2 comprenant en outre l'étape d'incorporation de ladite forme (S) du N-tert-butyl-2-pipérazinecarboxamide dans un inhibiteur de protéase de VIH.

4. Procédé selon l'une quelconque des revendications 1 à 3, dans lequel la résolution optique de l'étape c) est réalisée par un acide aminé N-acylé optiquement actif.

5. Procédé selon la revendication 4, dans lequel ledit acide aminé N-acylé optiquement actif est la N-benzyloxycarbonyl-L-phénylalanine ou l'acide N-benzyloxycarbonyl-L-aspartique.

6. 2-cyanopipérazine de formule (I) 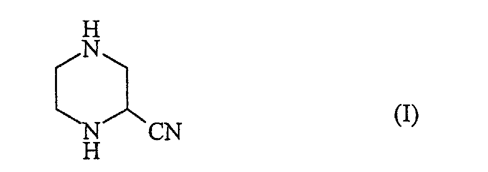 ou sel de celle-ci.

7. Procédé de préparation de la 2-cyanopipérazine de formule (I) définie dans la revendication 6, caractérisé en ce qu'un 2-halogénoacrylonitrile de formule (IX): 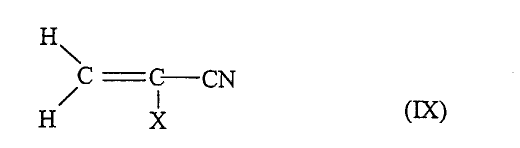 où X est un atome d'halogène, est mis à réagir avec l'éthylènediamine.

8. Procédé selon la revendication 7, dans lequel la proportion molaire de l'éthylénediamine par rapport au 2-halogénoacrylonitrile (IX) est d'environ 1,5:1.

9. Procédé selon la revendication 7 ou 8, dans lequel ledit 2-halogéno-acrylonitrile (IX) comprend le 2-chloroacrylonitrile.

10. Procédé de préparation de la 2-cyanopipérazine formule (I) définie dans la revendication 6, caractérisé en ce qu'un 2,3-dihalogénopropionitrile de formule (X) : 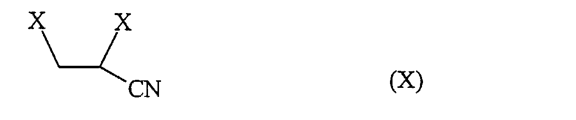 où X est un atome d'halogène, est mis à réagir avec l'éthylènediamine.

11. Procédé selon la revendication 10, dans lequel la proportion molaire de l'éthylènediamine par rapport au 2,3-dihalogénopropionitrile est d'environ 2 : 1.

12. Procédé selon la revendication 10 ou 11, dans lequel ledit 2,3-dihalogénopropionitrile (X) comprend le 2,3-dichloropropionitrile.

13. Procédé selon l'une quelconque des revendications 7 à 12, dans lequel la réaction est conduite en présence d'une base.

14. Procédé selon l'une quelconque des revendications 7 à 13, dans lequel la réaction est conduite en présence d'un solvant.

15. Utilisation de la 2-cyanopipérazine de formule (I) définie dans la revendication 6 pour la préparation du N-tert-butyl-2-pipérazinecarboxamide de formule (II) défini dans la revendication 1.

16. Utilisation de la 2-cyanopipérazine de formule (I) définie dans la revendication 6 pour la préparation d'une substance biologiquement active.

17. Utilisation de la 2-cyanopipérazine de formule (I) définie dans la revendication 6 pour la préparation d'un inhibiteur de protéase de VIH.
